# EUROPEAN PATENT APPLICATION

(11) **EP 4 647 440 A1**
(43) Date of publication of application: **12.11.2025**
(21) Application number: 23914966.9
(22) Date of filing: 08.12.2023
(51) Int. Cl.: C07K 14/47, A61K 39/00, A61P 35/00, A61K 38/00

(54) **TROP2 IMMUNOGENIC PEPTIDE**

(30) Priority: 04.01.2023 KR 20230001474
(71) Applicant: Aston Science Inc., Seoul 06193 (KR); Aston Sci. USA, Inc., Seattle, Washington 98105-4762 (US)
(72) Inventor: JUNG, Hun, Seoul 06193 (KR); PARK, Hyo Hyun, Hanam-si Gyeonggi-do 12925 (KR); CHOI, Jee Hyun, Hanam-si Gyeonggi-do 12925 (KR); KANG, Jin Ho, Hanam-si Gyeonggi-do 12925 (KR); LIM, Jin Back, Seoul 06193 (KR); JOUNG, Eun Kyo, Seoul 06193 (KR)
(74) Representative: Calysta NV
(86) International application number: PCT/KR2023/020268
(87) International publication number: WO 2024/147503

(57) **Abstract**

The present invention relates to TROP2 immunogenic peptides and a use thereof. The TROP2 immunogenic peptide according to the present invention selectively binds to MHC class II to enhance only the immunogenicity of specific immune cells capable of killing cancer cells and thus can be advantageously used as an excellent cancer vaccine for the prevention and/or treatment of cancer by minimizing the immune escape mechanism of cancer cells.

## Description

### [Technical Field]

The present invention relates to a novel tumor-associated calcium signal transducer 2 (TROP2) immunogenic peptide and a use thereof.

### [Background Art]

Cancer vaccines are designed to activate cytotoxic T cells, causing the activated T cells to recognize and act against a specific type of cancer, or to induce the production of antibodies that bind to antigens present on the surface of cancer cells. The basic therapeutic goal of cancer vaccines is to reduce the size of cancer tissue already present in a cancer patient, or to inhibit cancer recurrence after the removal of tumor tissue via surgery or chemotherapy in patients with cancer.

Among these cancer vaccines, HER2 cancer vaccines are the most widely researched and actively undergoing clinical trials. 'HER2' is a member of the epidermal growth factor receptor (EGFR) family (EGFR2), and overexpression of EGFR is observed in many cancer types. In particular, HER2 overexpression is observed in 15-30% of all breast cancer patients, and HER2 expression is known to be related to recurrence and poor prognosis in breast cancer patients.

HER2 anticancer vaccines have been extensively studied in breast cancer, and several groups are developing cancer vaccines using various immunogenic peptides; however, significant clinical outcomes have yet to be achieved.

As described above, one of the primary reasons for the limited clinical success is that previous therapeutic vaccines have predominantly focused on activating CD8⁺ cytotoxic T cells. This development direction is based on the idea that cytotoxic immunity plays a major role in anticancer responses. However, it has recently been reported that CD8⁺ T cell activation alone cannot induce sufficient immune responses if it is not accompanied by the activation of CD4⁺ Type 1 helper T cells (T-helper 1 cells; Th1 cells).

Therefore, it is important to design vaccines in a way that enables the induction of vaccine-specific Th1 immunity early in the vaccine development process.

TROP2 is a type 1 single-pass transmembrane protein consisting of 323 amino acid residues. TROP2 is known to be overexpressed on the surface of many types of epithelial cancer cells, such as prostate cancer, colorectal cancer, pancreatic cancer, and ovarian cancer, but the role of TROP2 protein in the growth and proliferation of cancer cells is not well understood.

However, since TROP2 protein shows an increased expression rate in many cancer types, attempts to produce anti-TROP2 antibodies as a therapeutic approach for cancer have been made, but have failed; instead, ADCs using anti-TROP2 antibodies have shown therapeutic effects and are being used for some cancer treatments under the name TRODELVY^{®} (sasituzumab govitecan). However, no research on immunogenic peptides for TROP2 has been known to date.

Under this technical background, the present inventors employed a partial sequence of the TROP2 protein as an immunogenic peptide, confirmed that it induces T cell-mediated immunity of specific T cells that recognize the protein as an antigen, and completed the present invention for the purpose of utilizing the part of the sequence as a cancer vaccine.

### [Disclosure]

### [Technical Problem]

An object of the present invention is to provide a novel tumor-associated calcium signal transducer 2 (TROP2) immunogenic peptide, a polynucleotide encoding the peptide, an immunogenic composition comprising the same, a use thereof, and a method of using the same.

Another object of the present invention is to provide a tumor-associated calcium signal transducer 2 (TROP2) immunogenic peptide.

Still another object of the present invention is to provide a polynucleotide encoding the TROP2 immunogenic peptide, a vector comprising the same, and/or a transformant modified to express the same.

Still another object of the present invention is to provide an immunogenic composition comprising the TROP2 immunogenic peptide, a polynucleotide encoding the same, a vector comprising the same, and/or a transformant modified to express the same.

Still another object of the present invention is to provide a pharmaceutical composition for preventing or treating cancer, comprising: the TROP2 immunogenic peptide, a polynucleotide encoding the same, a vector comprising the same, and/or a transformant modified to express the same.

Still another object of the present invention is to provide an anticancer vaccine composition comprising the TROP2 immunogenic peptide, a polynucleotide encoding the same, a vector comprising the same, and/or a transformant modified to express the same.

Still another object of the present invention is to provide a method for treating or preventing a disease, comprising: administering to a subject the TROP2 immunogenic peptide; the polynucleotide encoding the same, a vector comprising the same, and/or a transformant modified to express the same; or an immunogenic composition thereof.

Still another object of the present invention is to provide a pharmaceutical use of the TROP2 immunogenic peptide, the polynucleotide encoding the same, the vector comprising the same, and/or the transformant modified to express the same; or the immunogenic composition thereof.

### [Technical Solution]

The present invention will be described in detail as follows. Meanwhile, each description and embodiment disclosed in the present invention may be applied to each of the other descriptions and embodiments. In other words, all combinations of various elements disclosed in the present invention fall within the scope of the present invention. In addition, it cannot be considered that the scope of the present invention is limited by specific descriptions described below.

### TROP2 Immunogenic Peptide

In one general aspect, the present invention provides an immunogenic peptide, isolated from the TROP2 full-length peptide of SEQ ID NO: 1, having a sequence of 50 or fewer amino acids and comprising any one amino acid sequence selected from the group consisting of SEQ ID NO: 2 to SEQ ID NO: 27.

More specifically, the present invention provides an immunogenic peptide, isolated from the TROP2 full-length peptide of SEQ ID NO: 1, having a sequence of 50 or fewer amino acids and comprising or consisting of any one amino acid sequence selected from the group consisting of SEQ ID NO: 2 to SEQ ID NO: 27.

Tumor-associated calcium signal transducer 2 (TROP2), GA733-1, EGP-1, M1S1 (hereinafter, referred to as TROP2, SEQ ID NO: 1) is a type 1 single-pass transmembrane protein consisting of 323 amino acid residues. The SEQ ID NO: 1 is derived from Uniprot No. P09758.

The TROP2 immunogenic peptide of the present invention may preferably be a peptide having an amino acid sequence of 50 or less and having any one sequence selected from the group consisting of SEQ ID NOs: 3, 8, 10, 11, 15, 17, 18, 20, 22 and 24, and more preferably, may be a peptide having any one sequence selected from the group consisting of SEQ ID NOs: 3, 10, 17 and 18.

The immunogenic peptide according to the present invention increases an immune response to the peptide in a subject to which it is administered, wherein it is characterized by stimulating T cells.

Further, the peptide according to the present invention may be characterized by specifically binding to major histocompatibility complex II (MHC class II).

In addition, the peptide according to the present invention may be characterized by stimulating Type 1 helper T cells (Th1 cells).

In addition, the peptide according to the present invention may be characterized by not stimulating regulatory T cells (T-reg).

"Immunogenic peptide" is a substance that, when administered to an animal, including a human, is capable of inducing an immune response in the animal, and the terms "peptide" and "epitope (antigenic determinant)" are used interchangeably herein.

The immune response induced by the immunogenic peptide may be cellular (T-cell mediated) and/or humoral (B-cell mediated, antibody-producing) immune response.

Among the T cells involved in the immune response, CD8⁺ T cells are activated by binding to MHC class I-expressing cells via immunogenic peptides, and CD4⁺ T cells are activated by binding to MHC class II-expressing antigen presenting cells (APCs) via immunogenic peptides.

The Immunogenic peptides presented by MHC class I are typically short peptides of 8 to 11 amino acids in length, while MHC class II presents longer peptides, typically 13 or more amino acids in length. Immunogenic peptide-MHC class II complexes are presented on the surface of antigen-presenting cells (APCs).

An "antigen" is a molecule or combination of molecules that is intended to induce an immune response to facilitate its recognition by the immune system of a living organism. The antigen may be a protein expressed by the host's cells, such as autoantigen, or tumor antigen. The antigen may be a complete protein or any part of a protein, such as an immunogenic peptide of a protein.

As used herein, the antigen may also be composed of a synthetic protein or molecule containing multiple immunogenic peptides (fusion polypeptides) in which several immunogenic peptides are linked.

The TROP2 immunogenic peptide according to the present invention may induce an immune response, preferably T cell-mediated immunity, against TROP2. In particular, the TROP2 immunogenic peptide according to the present invention may selectively stimulate Type 1 helper T cells (Th1 cells) capable of recognizing TROP2 as an antigen among T cells that may have immunogenicity, thereby inducing antigen-specific immunogenicity, while at the same time not stimulating regulatory T cells that inhibit immune responses, thereby directly or indirectly killing cancer cells.

Specifically, the present invention may act in a direction that increases the secretion of interferon gamma (IFN-γ) from Type 1 helper T cells activated by stimulation by the peptide of the present invention, and does not stimulate the secretion of interleukin 10 (IL-10), a substance that inhibits cytotoxicity against cancer cells. Cell presentation for Th1 cell stimulation is performed through MHC class II. Therefore, the peptide according to the present invention may exhibit excellent efficacy in that it acts by selectively binding to MHC class II.

Furthermore, unlike MHC class I, MHC class II has the characteristic that a reaction occurs between MHC class and T cell receptor even if it matches only a portion of the full epitope. In other words, the immunogenic peptide according to the present invention does not show HLA type restriction and thus has universality (off-the shelf).

Therefore, the peptide according to the present invention may exhibit an acting effect as an immunogenic peptide, which may be utilized for the purpose of a cancer vaccine or for the purpose of preventing or treating cancer.

The peptide according to the present invention may exhibit one or more of the following functional effects, but is not limited thereto:
an effect that selectively binds to MHC class II to induce CD4⁺ T cell response;
an effect that selectively stimulates Type 1 helper T cells (Th1 cells) to induce antigen-specific immunogenicity;
an effect that stimulates Type 1 helper T cells (Th1 cells) to stimulate the secretion of interferon gamma (IFN-γ), which can directly or indirectly kill cancer cells, and
that does not stimulate the secretion of interleukin 10 (IL-10);
an effect that does not stimulate regulatory T cells;
an effect that does not stimulate Type 2 helper T cells (Th2 cells);
an effect that increases immunogenicity of CD8⁺ T cells;
an effect that activates memory T cells as well as short-term immunogenicity;
an epitope spreading effect of different cancer-related epitopes derived from cancer tissue;
an effect that inhibits tumor recurrence or progression by increasing the immunogenicity of Type 1 helper T cells (Th1 cells); and
an effect that increases NK cells in addition to T cells.

Any one of the peptides selected from the group consisting of SEQ ID NO: 2 to SEQ ID NO: 27 according to the present invention may also be described based on the sequence of the TROP2 full-length sequence of SEQ ID NO: 1, which is summarized in Table 1 below:

**[Table 1]**

| **SEQ ID NOs:** | **Numbering based on SEQ ID NO: 1 (based on full-length sequence)** | **Peptide Sequences** |
|---|---|---|
| 2 | 9 - 23 | PPPLRLPLLLLVLAA |
| 3 | 11 - 32 | PLRLPLLLLVLAAVTGHTAAQD |
| 4 | 15 - 29 | PLLLLVLAAVTGHTA |
| 5 | 19 - 33 | LVLAAVTGHTAAQDN |
| 6 | 67 - 81 | STLTSKCLLLKARMS |
| 7 | 70 - 84 | TSKCLLLKARMSAPK |
| 8 | 71 - 85 | SKCLLLKARMSAPKN |
| 9 | 73 - 87 | CLLLKARMSAPKNAR |
| 10 | 89 - 103 | LVRPSEHALVDNDGL |
| 11 | 142 - 168 | SLRCDELVRTHHILIDLRHRPTAGAFN |
| 12 | 146 - 160 | DELVRTHHILIDLRH |
| 13 | 152 - 166 | HHILIDLRHRPTAGA |
| 14 | 179 - 193 | LFRERYRLHPKFVAA |
| 15 | 181 - 195 | RERYRLHPKFVAAVH |
| 16 | 182 - 196 | ERYRLHPKFVAAVHY |
| 17 | 204 - 218 | ELRQNTSQKAAGDVD |
| 18 | 247 - 261 | RGEPLQVERTLIYYL |
| 19 | 249 - 263 | EPLQVERTLIYYLDE |
| 20 | 256 - 270 | TLIYYLDEIPPKFSM |
| 21 | 264 - 278 | IPPKFSMKRLTAGLI |
| 22 | 266 - 282 | PKFSMKRLTAGLIAVIV |
| 23 | 267 - 281 | KFSMKRLTAGLIAVI |
| 24 | 275 - 308 | AGLIAVIVVVVVALVAGMAVLVITNRRK SGKYKK |
| 25 | 282 - 296 | VVVVVALVAGMAVLV |
| 26 | 291 - 305 | GMAVLVITNRRKSGK |
| 27 | 295 - 309 | LVITNRRKSGKYKKV |

The numbering described in Table 1 above refers to the amino acid numbering based on the TROP2 full-length peptide sequence of SEQ ID NO: 1.

The peptide according to the present invention having an amino acid sequence of 50 or less means that the number of consecutive amino acid sequences is at most 50 or fewer. It preferably means having an amino acid sequence of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50. More preferably, it may comprise or consist of a sequence of 15 to 34 consecutive amino acids.

More specifically, the polypeptide fragment may preferably comprise at least 10 consecutive amino acids of a region defined by amino acid positions 9 to 23 of SEQ ID NO: 1 (SEQ ID NO: 2).

For example, identifying SEQ ID NO: 2 (the amino acid sequence from position 9 to 23 according to the sequence numbering based on SEQ ID NO: 1) may further comprise an amino acid sequence of the N-terminus and/or C-terminus of the 15 amino acid sequence. More specifically, the peptide may be an immunogenic peptide, isolated from the TROP2 full-length peptide of SEQ ID NO: 1, which may be configured to have an amino acid sequence of at most 50 amino acids based on SEQ ID NO: 2, and thus further comprise at least 1, 2, 3, 4, 5, 6, 7, 8, 9, 10, 11, 12, 13, 14, 15, 16, 17, 18, 19, or 20 amino acids at the N-terminus and/or C-terminus of the Core sequence (the amino acid sequence from position 9 to 23 based on SEQ ID NO: 1).

As described above, the immunogenic peptide of the present invention may be an isolated immunogenic peptide of the TROP2 full-length peptide of SEQ ID NO: 1, which further comprises the amino acid sequence of the N-terminus and/or the C-terminus, up to a maximum number of amino acid sequences of 50 or less even with respect to SEQ ID NOs: 3 to 27.

As described in the SEQ ID NO: 2 above, the polypeptide fragment may be an isolated immunogenic peptide of the TROP2 full-length peptide of SEQ ID NOS: 3 to 27, comprising at least 10 consecutive amino acids of a region defined by the amino acid positions according to the sequence numbering based on SEQ ID NO: 1 as summarized in Table 1 above.

In addition, the peptide may be a peptide having at least 90% homology with any one amino acid sequence selected from the group consisting of SEQ ID NOS: 2 to 27. More specifically, the homology refers to a peptide having 90%, 91%, 92%, 93%, 94%, 95%, 96%, 97%, 98%, 99%, or higher sequence homology. The sequence within the homology range may comprise a sequence having any one or more amino acid sequence (core sequence) selected from the group consisting of SEQ ID NOs: 2 to 27 and having sequence homology within the range of an isolated immunogenic peptide of the TROP2 full-length peptide of SEQ ID NO: 1.

More specifically, the peptide may be a sequence having functional homology to a peptide having at least one amino acid deletion, insertion and/or substitution in any one of the sequences selected from the group consisting of the above-described SEQ ID NO: 2 to SEQ ID NO: 27. The functional homology may mean exhibiting the efficacy of immunogenicity desired in the present invention.

The amino acid sequence may be modified by, for example, 1, 2, 3, 4, or 5 (i.e., up to 5) additions, deletions or substitutions, provided that, compared to a peptide having an unmodified sequence, the polypeptide having the modified sequence may exhibit the same or increased immunogenicity against TROP2. The term "the same" should be understood to mean that the peptide of the modified sequence does not exhibit significantly reduced immunogenicity compared to the peptide of the unmodified sequence.

If necessary, any one or more peptides selected from the group consisting of SEQ ID NO: 2 to SEQ ID NO: 27 according to the present invention may be linked together directly or via a linker at the N-terminus or C-terminus. The any one or more peptides may be two or more peptides that are identical or different.

In other words, in another general aspect, the present invention also provides a fusion polypeptide comprising at least one peptide selected from the group consisting of SEQ ID NO: 2 to SEQ ID NO: 27; and a linker.

The phrase "linked" according to the present invention refers to the linkage and/or binding of a peptide sequence to an N-terminus or C-terminus. Here, the linkage includes both direct bonding and connection via a linker or spacer peptide.

The term "linker" or "spacer" refers to a short amino acid sequence used to separate two peptides with different functions in the construction of the fusion polypeptide. The absence of a linker between two or more individual domains in a protein may result in reduced or inappropriate function of the protein domain due to steric hindrance, for example, reduced binding affinity for MHC class II or T cell receptors. In the present invention, the space between each peptide may be increased when an artificial linker is used to connect each peptide in a fusion protein. The linker or spacer may have a form commonly used in the art.

### Polynucleotide encoding TROP2 immunogenic peptide, plasmid vector comprising the same, and/or transformant modified to express the same

The present invention provides a polynucleotide encoding an immunogenic peptide and/or a fusion polypeptide thereof, the immunogenic peptide being isolated from the TROP2 full-length peptide of SEQ ID NO: 1, having a sequence of 50 or fewer amino acids and comprising any one amino acid sequence selected from the group consisting of SEQ ID NO: 2 to SEQ ID NO: 27.

The present invention also provides a polynucleotide encoding an immunogenic peptide and/or a fusion polypeptide thereof, the immunogenic peptide being isolated from the TROP2 full-length peptide of SEQ ID NO: 1, having a sequence of 50 or fewer amino acids and comprising or consisting of any one amino acid sequence selected from the group consisting of SEQ ID NO: 2 to SEQ ID NO: 27.

The polynucleotide may be DNA, mRNA, or a commonly available modified nucleotide.

In addition, when the form of the polynucleotide is mRNA, it may be chemically synthesized or synthesized in vitro by RNA polymerase.

The mRNA molecule may further comprise one or more selected from the group consisting of a 5'-cap, a 5'-untranslated region (UTR), a 3'-untranslated region, and a poly(A) tail. If necessary, the mRNA may each comprise a cap structure or a modified cap structure.

In addition, the mRNA molecule may be composed of a nucleic acid including adenine (A), thymine (T), guanine (G), cytosine (C), uridine (U), and/or modified forms thereof.

In another aspect, the present invention provides a vector comprising the polynucleotide.

Preferably, the vector may be a plasmid vector, a viral vector, or a virus-like particle, and the vector comprising the polynucleotide may be produced by a recombinant technique according to a genetic engineering method.

In still another general aspect, the present invention provides a transformant transformed with an expression vector comprising the polynucleotide.

Cells (transformants) transformed with the expression vector of the present invention may have a therapeutic effect by being administered to a subject.

### Immunogenic composition comprising TROP2 immunogenic peptide, polynucleotide encoding the same, vector comprising the same, and/or transformant modified to express the same

### Immunogenic composition

The present invention provides an immunogenic composition comprising the peptide, the fusion polypeptide, the polynucleotide encoding the same, the vector comprising the same, and/or the transformant modified to express the same according to the present invention.

Further, the present invention provides an immunogenic composition comprising one or more peptides, fusion polypeptides, polynucleotides encoding the same, vectors comprising the same, and/or transformants modified to express the same according to the present invention; and optionally one or more adjuvants, pharmaceutically acceptable carriers, preservatives, and/or excipients.

The immunogenic composition may comprise, for example, at least one, two, three, four, five, six, seven, eight or more different peptides or fusion polypeptides of the present invention; and optionally at least one adjuvant, pharmaceutically acceptable carrier, preservative, and/or excipient.

The immunogenic composition may comprise a polynucleotide encoding at least one, two, three, four, five, six, seven, eight or more different peptides or fusion polypeptides of the present invention; and optionally at least one adjuvant, pharmaceutically acceptable carrier, preservative, and/or excipient.

The carrier, preservative, and excipient refer to ingredients that are compatible with other components of the composition and suitable for pharmaceutically use.

The immunogenic composition of the present invention may also preferably further comprise an adjuvant. The adjuvant is any substance that, when included in a composition, enhances or otherwise modifies the immune response induced by the composition. An adjuvant, broadly defined, is a substance that stimulates the immune response. The adjuvant is classified into three types based on its mechanism: i) for antigen delivery, ii) for enhancing immune response, and iii) for a combination of these functions. Representative adjuvants for antigen delivery include liposomes and Montanide emulsion (IFA; Incomplete Freund's adjuvant), and representative adjuvants for enhancing immune response include granulocyte-macrophage colony-stimulating factor (GM-CSF) and toll-like receptor agonists (TLR agonists).

The adjuvant, regardless of its mechanism, may be selected from one or more of the following groups: AlK(SO₄)₂, AlNa(SO₄)₂, AlNH₄ (SO₄), silica, alum, Al(OH)₃, Ca₃(PO₄)₂, kaolin, carbon, aluminum hydroxide, muramyl dipeptides, N-acetyl-muramyl-L-threonyl-D-isoglutamine (thr-DMP), N-acetyl-nor-muramyl-L-alanyl-D-isoglutamine (CGP 11687, also known as nor-MDP), N-acetylmuramyl-L-alanyl-D-isoglutaminyl-L-alanine-2-(1'2'-dipalmitoyl-sn-glycero-3-hydroxyphosphoryloxy)-ethylamine (CGP 19835A, also known as MTP-PE), RIBI (MPL + TDM + CWS) in 2% squalene/Tween-80^{™} emulsion, lipopolysaccharides and its various derivatives including lipid A, Freund's Complete Adjuvant (FCA), Freund's Incomplete Adjuvants, Merck Adjuvant 65, polynucleotides (e.g., poly IC and poly AU acids), wax D of Mycobacterium tuberculosis, substances found in Corynebacterium parvum, Bordetella pertussis, and members of the genus Brucella, TiterMax, ISCOMs, Quil A, ALUN (see U.S. Patent Nos. 58767 and 5,554,372), lipid A derivatives, choleratoxin derivatives, HSP derivatives, LPS derivatives, synthetic peptide matrices or GMDP, interleukin 1, interleukin 2, Montanide ISA-51, and QS-21, various saponin extracts, GM-CSF, TLR agonists, etc., may be used as adjuvants.

When a composition includes an excipient, the excipient should be "pharmaceutically acceptable" in the sense that it is compatible with the other ingredients of the composition and is not harmful to its recipient.

### Pharmaceutical composition

The present invention provides a pharmaceutical composition for preventing or treating cancer comprising: the TROP2 immunogenic peptide, a polynucleotide encoding the same, a vector comprising the same, and/or a transformant modified to express the same.

The present invention also provides a pharmaceutical composition for preventing or treating cancer comprising one or more peptides, fusion polypeptides, polynucleotides encoding the same, vectors comprising the same, and/or transformants modified to express the same according to the present invention; and optionally one or more adjuvants.

Further, the present invention provides a pharmaceutical composition for preventing or treating cancer comprising one or more peptides, fusion polypeptides, polynucleotides encoding the same, vectors comprising the same, and/or transformants modified to express the same according to the present invention; and optionally one or more adjuvants, pharmaceutically acceptable carriers, preservatives, and/or excipients.

The pharmaceutical composition may comprise, for example, at least one, two, three, four, five, six, seven, eight or more different peptides or fusion polypeptides of the present invention; and optionally at least one adjuvant, pharmaceutically acceptable carrier, preservative, and/or excipient.

Further, the pharmaceutical composition may comprise a polynucleotide encoding at least one, two, three, four, five, six, seven, eight or more different peptides or fusion polypeptides of the present invention; and optionally at least one adjuvant, pharmaceutically acceptable carrier, preservative, and/or excipient.

The carrier, preservative, and excipient refer to ingredients that are compatible with other components of the composition and suitable for pharmaceutically use.

The description of the excipients and adjuvants in the pharmaceutical composition is as described above.

The pharmaceutical composition may further comprise an additional cancer therapeutic agent.

As the additional cancer therapeutic agent, an antibody-drug conjugate is preferable from the viewpoint of the synergistic effect of the antitumor effect when used in combination with the TROP2 immunogenic peptide of the present invention. More specifically, the additional cancer therapeutic agent may be trastuzumab deruxtecan (Enhertu), sacituzumab govitecan (Trodelvy), brentuximab vedotin (Adcetris), trastuzumab emtansine (Kadcyla), polatuzumab vedotin (Polivy), inotuzumab ozogamicin (Besponsa), gemtuzumab ozogamicin (Mylotarg), and moxetumomab pasudotox (Lumoxiti), and the preferable additional cancer therapeutic agent may be sacituzumab govitecan (Trodelvy).

The cancer may be any one selected from the group consisting of breast cancer, brain cancer, nerve cancer, colorectal cancer, lung cancer, non-small cell lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, perianal cancer, colon cancer, breast cancer, fallopian tube carcinoma, endometrial carcinoma, cervical cancer, vaginal cancer, vulvar carcinoma, Hodgkin's disease, esophageal cancer, small intestinal cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary CNS lymphoma, spinal cord tumor, brainstem glioma, and pituitary adenoma.

Specifically, the cancer may be a cancer that expresses TROP2, or a cancer that is responsive to existing targeted therapeutic agents for TROP2 (such as Trodelvy^{®}).

Further, the cancer may be colorectal cancer, esophageal cancer, gallbladder cancer, bile duct cancer, pancreatic cancer, ovarian cancer, head and neck cancer, breast cancer, non-small cell lung cancer, or endometrial cancer.

### Anticancer vaccine composition

The present invention provides an anticancer vaccine composition comprising a TROP2 immunogenic peptide, a polynucleotide encoding the same, a vector comprising the same, and/or a transformant modified to express the same.

The present invention also provides an anticancer vaccine composition comprising one or more peptides, fusion polypeptides, polynucleotides encoding the same, vectors comprising the same, and/or transformants modified to express the same according to the present invention; and optionally one or more adjuvants.

Further, the present invention provides an anticancer vaccine composition comprising one or more peptides, fusion polypeptides, polynucleotides encoding the same, vectors comprising the same, and/or transformants modified to express the same according to the present invention; and optionally one or more adjuvants, pharmaceutically acceptable carriers, preservatives, and/or excipients.

The anticancer vaccine composition may comprise, for example, at least one, two, three, four, five, six, seven, eight or more different peptides or fusion polypeptides of the present invention; and optionally at least one adjuvant, pharmaceutically acceptable carrier, preservative, and/or excipient.

Further, the anticancer vaccine composition may comprise a polynucleotide encoding at least one, two, three, four, five, six, seven, eight or more different peptides or fusion polypeptides of the present invention; and optionally at least one adjuvant, pharmaceutically acceptable carrier, preservative, and/or excipient.

The anticancer vaccine composition according to the present invention is preferably a cancer vaccine composition containing a TROP2 immunogenic peptide.

However, in one feasible embodiment of the vaccine composition, the vaccine may also be utilized in the form of a DNA vaccine, an mRNA vaccine, an adenovirus vaccine, or other viral vector vaccines.

In addition, the vaccine composition may be utilized in the form of immune effector cells transformed with a vector comprising a polynucleotide encoding the immunogenic peptide.

The carrier, preservative, and excipient refer to ingredients that are compatible with other components of the composition and suitable for pharmaceutically use.

The description of "excipient" and "adjuvant" in the anticancer vaccine composition is as described above.

In addition, the description of "cancer" in the anticancer vaccine composition is as described above.

The composition of the present invention may be provided in the form of a kit including instructions, etc.

For example, the kit of the present invention may include a cancer vaccine composition and instructions, etc. In addition, one or more adjuvants may be included in the kit to increase the immunogenicity of the composition.

### Method of using TROP2 immunogenic peptide, the polynucleotide encoding the same, the vector comprising the same, and/or the transformant modified to express the same; or the compositions thereof and pharmaceutical use thereof

The present invention provides a method for treating or preventing a disease or condition, comprising: administering to a subject the TROP2 immunogenic peptide, the fusion polypeptide, the polynucleotide encoding the same, the vector comprising the same, and/or the transformant modified to express the same; or the composition thereof.

The present invention provides the use in the manufacture of a medicament for treating or preventing a disease or condition, of the TROP2 immunogenic peptide, the fusion polypeptide, the polynucleotide encoding the same, the vector comprising the same, and/or the transformant modified to express the same; or the composition thereof.

The method comprises administering to a subject the TROP2 immunogenic peptide, the fusion polypeptide, the polynucleotide encoding the same, the vector comprising the same, and/or the transformant modified to express the same; or the composition thereof.

With respect to administration, to a subject in need thereof, a therapeutically or prophylactically effective amount of the TROP2 immunogenic peptide, the fusion polypeptide, the polynucleotide encoding the same, the vector comprising the same, and/or the transgene modified to express the same; or the composition thereof, may be administered.

The disease or condition may refer to a disease or condition capable of being prevented or treated by selectively binding to MHC class II and stimulating CD4⁺ T cells, and in particular, stimulating Type 1 helper T cells, and not stimulating T-reg cells, through the TROP2 immunogenic peptide, the fusion polypeptide, the polynucleotide encoding the same, the vector comprising the same, and/or the transformant modified to express the same; or the composition thereof.

More specifically, the disease or condition may mean a disease or condition capable of being prevented or treated by stimulating the secretion of interferon gamma (IFN-gamma) and not stimulating the secretion of interleukin 10 (IL-10) through the TROP2 immunogenic peptide, the polynucleotide encoding the same, the vector comprising the same, and/or the transformant modified to express the same; or the immunogenic composition thereof.

More specifically, the disease or condition is cancer.

The cancer may be any one selected from the group consisting of breast cancer, brain cancer, nerve cancer, colorectal cancer, lung cancer, non-small cell lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, perianal cancer, colon cancer, breast cancer, fallopian tube carcinoma, endometrial carcinoma, cervical cancer, vaginal cancer, vulvar carcinoma, Hodgkin's disease, esophageal cancer, small intestinal cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary CNS lymphoma, spinal cord tumor, brainstem glioma, and pituitary adenoma.

Specifically, the cancer may be a cancer that expresses TROP2, or a cancer that is responsive to existing targeted therapeutic agents for TROP2 (such as Trodelvy^{®}).

Further, the cancer may be colorectal cancer, esophageal cancer, gallbladder cancer, bile duct cancer, pancreatic cancer, ovarian cancer, head and neck cancer, breast cancer, non-small cell lung cancer, or endometrial cancer.

Thus, the present invention provides a method for treating or preventing cancer, comprising: administering to a subject the TROP2 immunogenic peptide, the fusion polypeptide, the polynucleotide encoding the same, the vector comprising the same, and/or the transformant modified to express the same; or the composition thereof.

The present invention provides the use in the manufacture of a medicament for treating or preventing cancer, of the TROP2 immunogenic peptide, the fusion polypeptide, the polynucleotide encoding the same, the vector comprising the same, and/or the transformant modified to express the same; or the composition thereof.

The present invention provides the TROP2 immunogenic peptide, the fusion polypeptide, the polynucleotide encoding the same, the vector comprising the same, and/or the transformant modified to express the same; or the composition thereof, for treating or preventing cancer.

The present invention provides the use in the manufacture of an anticancer vaccine, of the TROP2 immunogenic peptide, the fusion polypeptide, the polynucleotide encoding the same, the vector comprising the same, and/or the transformant modified to express the same; or the immunogenic composition thereof.

The present invention provides the TROP2 immunogenic peptide, the fusion polypeptide, the polynucleotide encoding the same, the vector comprising the same, and/or the transformant modified to express the same; or the immunogenic composition thereof, for use as a cancer vaccine.

The vaccine composition or pharmaceutical composition according to the present invention may be used in mammals, preferably humans, and may deliver the target substance to antigen presenting cells (APCs) in vivo, by injection via routes such as intravenous, intraperitoneal, intramuscular, subcutaneous, intradermal, nasal, mucosal, inhalation, intra-lymph node, and intra-tumor tissue routes.

The vaccine composition or pharmaceutical composition may be formulated and provided in an appropriate form.

The dosage of the composition may vary depending on the patient's age, weight, sex, dosage form, health status, and disease severity, and may be administered once a day or several times a day at regular intervals as determined by the physician or pharmacist.

The composition is administered to the subject at a set cycle. If necessary, a booster shot is administered.

The subject to which the composition is administered is preferably a mammal such as a human.

In addition, if necessary, the therapeutic effect may be enhanced through simultaneous or sequential administration with additional cancer treatment.

The additional cancer treatment may be selected from the following:
i) standard treatment for each cancer type,
ii) cytotoxic chemotherapeutic agents,
iii) small-molecule targeted therapeutic agents (olaparib, gefitinib, erlotinib, lafatinib, sunitinib, imatinib, crizotinib, etc.),
iv) monoclonal targeted therapeutic agents,
v) antibody-drug conjugates,
vi) other immunotherapeutic agents,
vii) topical treatment, and
viii) other therapeutic agents.

Therefore, the pharmaceutical composition of the present invention may be administered simultaneously with the treatments i) to viii) or sequentially at regular intervals.

As a specific additional cancer therapeutic agent, an antibody-drug conjugate is preferable from the viewpoint of the synergistic effect of the antitumor effect when used in combination with the TROP2 immunogenic peptide of the present invention. More specifically, the additional cancer therapeutic agent is preferably at least one selected from the group consisting of trastuzumab deruxtecan (Enhertu), sacituzumab govitecan (Trodelvy), brentuximab vedotin (Adcetris), trastuzumab emtansine (Kadcyla), polatuzumab vedotin (Polivy), inotuzumab ozogamicin (Besponsa), gemtuzumab ozogamicin (Mylotarg), and moxetumomab pasudotox (Lumoxiti), and more preferably, sacituzumab govitecan (Trodelvy).

In an embodiment, the TROP2 immunogenic peptide of the present invention exhibits a significantly superior antitumor effect due to the synergistic effect when administered with a lower amount of ADC than a dose or standard dose generally used in combination therapy with an antibody-drug conjugate (ADC) comprising an antibody against TROP2.

Therefore, the combined administration of the TROP2 immunogenic peptide and the ADC according to the present invention exhibits the effect of reducing the amount of toxic ADC while maintaining or improving its therapeutic effect.

The TROP2 immunogenic peptide, the fusion polypeptide, the polynucleotide encoding the same, the vector comprising the same, and/or the transformant modified to express the same; or the composition thereof according to the present invention may enhance cancer treatment effects while reducing the therapeutic dose of a drug to show therapeutic efficacy when used in combination with any of the above-described drugs.

The present invention provides a method for stimulating TROP2-specific T cells in a subject, comprising: administering to a subject the TROP2 immunogenic peptide, the fusion polypeptide, the polynucleotide encoding the same, the vector comprising the same, and/or the transformant modified to express the same; or the composition thereof. More specifically, the stimulation of the T cells may be to stimulate Type 1 helper T cells (Th1 cells). In addition, it may also mean stimulating Type 1 helper T cells (Th1 cells) without stimulating regulatory T cells (T-reg).

The present invention also provides a method for providing information on immunogenicity against cancer, comprising: treating a TROP2 immunogenic peptide, a fusion polypeptide, a polynucleotide encoding the same, a vector comprising the same, and/or a transformant modified to express the same; or a composition thereof; and

measuring the level of stimulation of at least one selected from the group consisting of stimulation of Type 1 helper T cells (CD4⁺IFN-γ⁺), stimulation of Type 2 helper T cells (CD4⁺IL-4⁺), stimulation of interleukin 10 (IL-10)-producing regulatory T cells (CD4⁺IL-10⁺), and stimulation of regulatory T cells (CD4⁺CD25⁺Foxp3⁺), for providing information on immunogenicity against cancer.

A case where treatment with the TROP2 immunogenic peptide, the fusion polypeptide, the polynucleotide encoding the same, the vector comprising the same, and/or the transformant modified to express the same; or the composition thereof, stimulates Type 1 helper T cells (CD4⁺IFN-γ⁺), a case where the treatment dose not stimulate Type 2 helper T cells (CD4⁺IL-4⁺), a case where the treatment does not stimulate interleukin 10 (IL-10)-producing regulatory T cells (CD4⁺IL-10⁺), and/or a case where the treatment does not stimulate regulatory T cells (CD4⁺CD25⁺Foxp3⁺) is evaluated as having high immunogenicity for cancer.

The present invention also provides a method for providing information on immunogenicity against cancer, comprising: treating a TROP2 immunogenic peptide, a polynucleotide encoding the same, a vector comprising the same, and/or a transformant modified to express the same; or a composition thereof, and measuring the secretion level of IFN-γ, IL-10, or both, for providing information on immunogenicity against cancer.

A case where treatment with the TROP2 immunogenic peptide, the polynucleotide encoding the same, the vector comprising the same, and/or the transformant modified to express the same; or the composition thereof stimulates the secretion of IFN-γ and/or does not stimulate the secretion of IL-10 is evaluated as having high immunogenicity against cancer.

### [Advantageous Effects]

The TROP2 immunogenic peptide according to the present invention selectively binds to MHC class II to enhance only the immunogenicity of specific immune cells capable of killing cancer cells and thus can be advantageously used as an excellent cancer vaccine for the prevention and/or treatment of cancer by minimizing the immune escape mechanism of cancer cells.

In particular, the TROP2 immunogenic peptide according to the present invention induces T cell-mediated immunity, selectively stimulates Type 1 helper T cells (Th1 cells) capable of recognizing TROP2 as an antigen among T cells capable of having immunogenicity, and does not stimulate Type 2 helper T cells (Th2 cells) and regulatory T cells, thereby inducing antigen-specific immunogenicity. Through this, the TROP2 immunogenic peptide according to the present invention may exhibit excellent cancer vaccine efficacy by promoting the secretion of interferon gamma (IFN-gamma), which can directly or indirectly kill cancer cells, and not stimulating the secretion of interleukin 10 (IL-10), a substance that inhibits the function of immune cells around cancer cells.

Moreover, the TROP2 immunogenic peptide has the advantage of increasing the immunogenicity of not only CD4⁺ T cells but also CD8⁺ T cells, and not only increasing short-term immunogenicity but also stimulating memory immune cells (memory T cells) that react to the TROP2 antigen, leading to long-term preventive and therapeutic efficacy.

In addition, since the TROP2 immunogenic peptide specific for Type 1 helper T cells (Th1 cells) is used, vaccination (immunization) is expected to induce epitope spreading of different cancer-related epitopes in cancer tissues, and this increase in immunogenicity may inhibit tumor recurrence or progression, and have high utility value as a vaccine alone or in combination with other cancer therapeutic agents.

Lastly, the TROP2 immunogenic peptide according to the present invention has significant potential as a cancer vaccine composition because it is also effective in increasing NK cells in addition to T cells.

### [Description of Drawings]

FIG. 1 represents the flow cytometry plot results showing the phenotype of Type 1 helper T cells (CD4⁺IFN-γ⁺) following TROP2 immunogenic peptide treatment according to the present invention.
FIG. 2 represents the flow cytometry plot results showing the phenotype of Type 2 helper T cells (CD4⁺IL-4⁺) following TROP2 immunogenic peptide treatment according to the present invention.
FIG. 3 represents the flow cytometry plot results showing the phenotype of regulatory T cells (CD4⁺CD25⁺Foxp3⁺) following TROP2 immunogenic peptide treatment according to the present invention.
FIG. 4 represents the flow cytometry plot results showing the phenotype of interleukin 10 (IL-10)-producing regulatory T cells (CD4⁺IL-10⁺) following TROP2 immunogenic peptide treatment according to the present invention.
FIG. 5 is a graph showing the levels of TROP2-specific IFN-γ and IL-10 T cell spots by ELISpot analysis using PBMCs.
FIG. 6 is a graph showing the change in tumor size to confirm the antitumor effect of the immunogenic peptide (TROP2 peptide) according to the present invention in a Xenograft cancer mouse model.
FIG. 7 is a graph showing the change in tumor size to confirm the antitumor effect of the immunogenic peptide (TROP2 peptide) according to the present invention in combination with Trodelvy in a Xenograft cancer mouse model.

### [Best Mode]

Hereinafter, the present invention will be described in more detail through Examples. These Examples are only provided for illustrating the present invention in more detail, and it will be apparent to those skilled in the art that the scope of the present invention is not limited by these Examples according to the gist of the present invention.

### Example 1. Prediction of immunogenic peptides that form immunopeptidome with MHC class II

The Aston epitope prediction in-silico system (ASEP) program was used to predict immunogenic peptides in the TROP2 peptide full-length sequence that have strong potential to bind to MHC class II and form an immunopeptidome.

Specifically, the prediction model in the ASEP program was utilized through the integration and cross-validation of Consensus, SMM, NetMHCII-pan, Combinatorial, etc., and the ASEP scoring was performed using custom-developed calculation algorithm. The amino acid sequences with high scores were set as immunogenic peptide candidates, and the core sequence locations and ranges of the immunogenic peptide candidates were cross-validated with commercial programs to secure a group of MHC class II selective immunogenic peptide candidates.

This resulted in immunogenic peptides with 15 to 34 amino acid sequences from the TROP2 full-length sequence (SEQ ID NO: 1), as shown in Table 1 above.

The peptide sequences identified through the ASEP program were synthesized and used in the following Examples.

### Example 2. Confirmation of Type 1 helper T cell-specific immunogenic peptides through FACS analysis

The efficacy of individual immunogenic peptide candidates was evaluated through FACS analysis.

To this end, the degree of stimulation of Type 1 helper T cells (CD4⁺IFN-γ⁺), stimulation of interleukin 10 (IL-10)-producing regulatory T cells (CD4⁺IL-10⁺), stimulation of regulatory T cells (CD4⁺CD25⁺Foxp3⁺), and stimulation of Type 2 helper T cells secreting IL-4 and Th17 cells secreting IL-17 according to the treatment with the immunogenic peptide were confirmed.

After that, the results were organized according to the evaluation scoring criteria for selecting TROP2 immunogenic peptides.

### (1) Experimental method

### - Cell surface marker antigens, cell lines, and reagents

Human antibody anti-CD3 (clone HIT), BV421-conjugated anti-CD4 (clone RPA-T4), PE-Cy5-conjugated anti-CD25 (clone 2A3), PerCP-Cy5.5-conjugated anti-IFN-γ (clone 4S.B3), PE-conjugated anti-IL-10 (clone JES3-19F1), protein transport inhibitor (GolgiPlug), and stain buffer were purchased from BD Biosciences. Brilliant Violet 510-conjugated anti-IL-4 (clone MP4-25D2) was purchased from BioLegend, Inc. eFlor780-conjugated Fixable Viability dye, FITC-conjugated anti-Foxp3 (clone PCH101) and Foxp3/Transcription buffer were purchased from Themo Fisher Scientific Inc. Phorbol 12-myristate 13-acetate and Ionomycin were purchased from Sigma. Peripheral Blood Mononuclear Cells (PBMCs) from healthy humans were purchased from ATCC.

### - Induction of peptide activity (immunogenicity) in vitro

Healthy human peripheral blood mononuclear cells (PBMCs; 2,000,000 cells per well) were cultured in 96x round bottom well plates in the presence or absence of anti-CD3 (clone HIT) and peptide at the indicated ratios.

Specifically, the peptide synthesized in Example 1 at a concentration of 10 µg/ml was cultured with 1 µg/ml of anti-CD3 in a 37°C incubator for 72 hours. The culture medium consisted of complete RPMI1640 medium supplemented with 10% fetal bovine serum, 10 mM HEPES, 50 µM β-ME, and penicillin/streptomycin/L-glutamine. After 72 hours of culture, the cells were stimulated with Phorbol 12-myristate 13-acetate (50 ng/mL) and Ionomycine (1 µg/mL), which are intracellular cytokine secretion stimulators. To prevent cytokine secretion into the culture medium, the cells were co-stimulated with GolgiPlug, a protein transport inhibitor, at a concentration of 1/100 diluted in PBS for 6 hours in a 37°C incubator. After stimulation, the cells were washed twice with PBS.

### - Flow Cytometry

To determine only the viable cells among the PBMC cells, the cells were labeled with eFlor780-conjugated Fixable Viability dye diluted 1 : 100 in PBS for 15 minutes at 4°C, and washed twice with stain buffer after 15 minutes. After washing, the cells were surface-labeled with BV421-conjugated anti-CD4 (clone RPA-T4) and PE-Cy5-conjugated anti-CD25 (clone 2A3) antibodies diluted 1 : 100 in stain buffer for 30 minutes at 4°C. Then, the cells labeled with cell surface proteins were washed twice with stain buffer. Next, to detect cytokines secreted intracellularly, the cells were fixed for 1 hour at room temperature with fixation buffer and then washed with permeabilization buffer. To enable antibodies to detect intracellular cytokines, the fixed cells were labeled overnight at 4°C with PerCP-Cy5.5-conjugated anti-IFN-g (clone 4S.B3), Brilliant Violet 510-conjugated anti-IL-4 (clone MP4-25D2), anti-Foxp3 (clone PCH101), and PE-conjugated anti-IL-10 (clone JES3-19F1) diluted 1 : 100 in permeabilization buffer. Then, the cytokine-labeled cells were washed twice with stain buffer, and cell flow assays were performed using an Attune NxT Acoustic Focusing Cytometer (Invitrogen). Data analysis was performed using Flowjo v10.7.1 software (FlowJo).

### - Evaluation scoring method using FACS results

The scoring for FACS results to select Th1 cell-specific immunogenic peptides was performed under the following conditions:
1) Calculate the difference between the distribution of IFN-γ secreting T cells and the distribution of IL-10 secretion (A value),
2) Calculate the difference between the distribution of IFN-γ secreting T cells and the Treg distribution (B value),
3) Use the formula "(A value x 0.8) + (B value x 0.2)" as the final combined scoring, and
4) Select peptides that show a specific numerical value or higher as TROP2 immunogenic peptides.

### (2) Experimental Results

The activity results of the immunogenic peptide candidate group were confirmed by first isolating CD4⁺ T cells (helper T cells) from PBMCs, and then classifying the isolated cells into Type 1 helper T cells (CD4⁺IFN-γ⁺), Type 2 helper T cells (CD4⁺IL-4⁺), regulatory T cells (CD4⁺CD25⁺Foxp3⁺), and interleukin-10 (IL-10)-producing regulatory T cells (CD4⁺IL-10⁺) and analyzing the results.

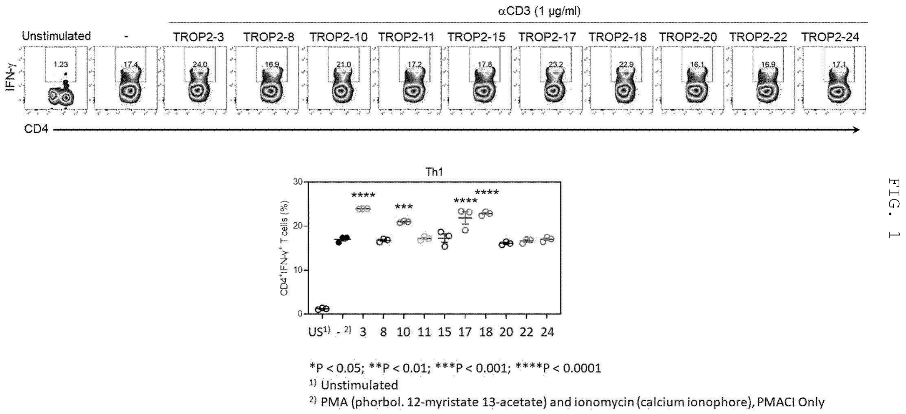

The results of each analysis are shown in FIGS. 1 to 4. FIGS. 1 to 4 represent the results of immunogenic peptides of SEQ ID NOs: 3, 8, 10, 11, 15, 17, 18, 20, 22, and 24, respectively.

FIG. 1 provides the flow cytometry plot results showing the phenotype of Type 1 helper T cells (CD4⁺IFN-γ⁺). As could be appreciated in FIG. 1, the treatment of the immunogenic peptide according to the present invention together with an intracellular cytokine secretion stimulator (Phorbol 12-myristate 13-acetate, ionomycin) resulted in a significant change in the phenotype of Type 1 helper T cells (CD4⁺IFN-γ⁺) . In particular, it could be confirmed that SEQ ID NOs: 3, 10, 17, and 18 stimulated Type 1 helper T cells (CD4⁺IFN-γ⁺) to a high degree.

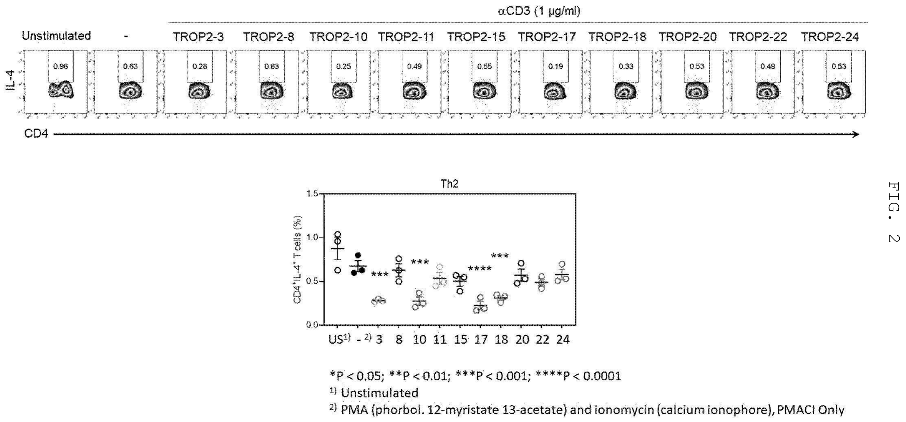

FIG. 2 provides the flow cytometry plot results showing the phenotype of Type 2 helper T cells (CD4⁺IL-4⁺). As could be appreciated in FIG. 2, the treatment of the immunogenic peptide according to the present invention together with an intracellular cytokine secretion stimulator (Phorbol 12-myristate 13-acetate, ionomycin) barely stimulated Type 2 helper T cells (CD4+IL-4+). In particular, the results showed that SEQ ID NOs: 3, 10, 17, and 18 hardly stimulated Type 2 helper T cells (CD4⁺IL-4⁺).

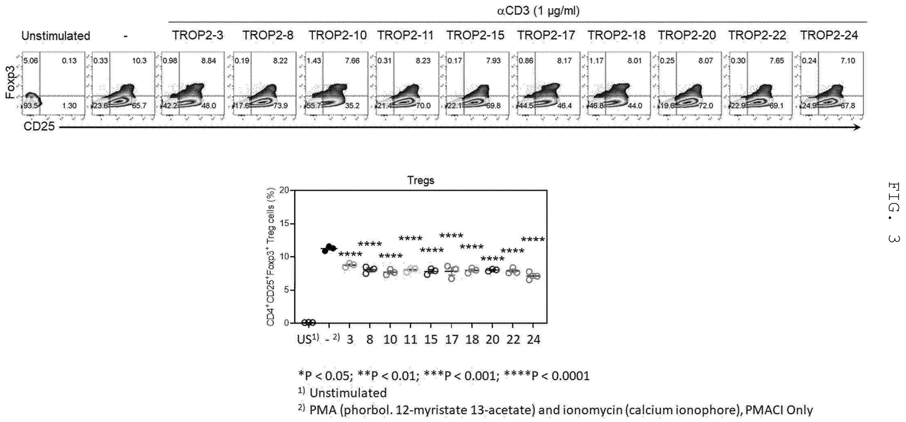

FIG. 3 provides the flow cytometry plot results showing the phenotype of regulatory T cells (CD4⁺CD25⁺Foxp3⁺). As could be appreciated in FIG. 3, the treatment of the immunogenic peptide according to the present invention together with an intracellular cytokine secretion stimulator (Phorbol 12-myristate 13-acetate, ionomycin) barely stimulated regulatory T cells (CD4⁺CD25⁺Foxp3⁺) .

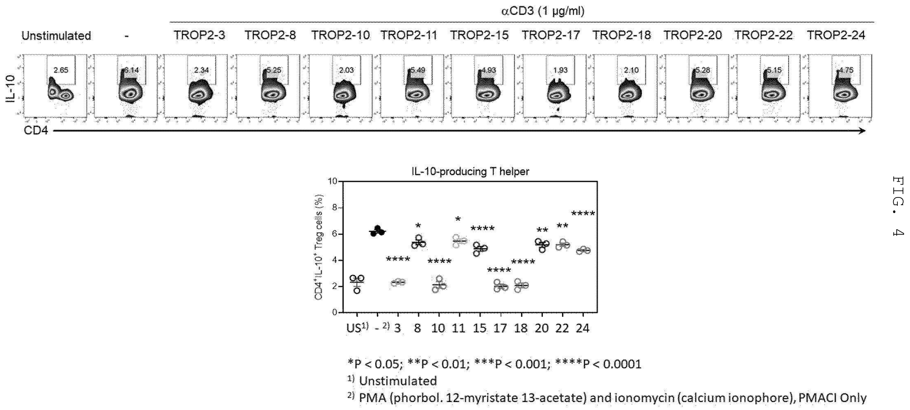

FIG. 4 provides the flow cytometry plot results showing the phenotype of interleukin 10 (IL-10)-producing regulatory T cells (CD4⁺IL-10⁺).

When the immunogenic peptide according to the present invention was treated together with an intracellular cytokine secretion stimulator (Phorbol 12-myristate 13-acetate, Ionomycine), it could be appreciated that it hardly stimulated interleukin 10 (IL-10)-producing regulatory T cells (CD4⁺IL-10⁺), similar to the results in FIG. 3.

Based on the above results, the calculation results of the FACS result values are shown in Table 2 below.

**[Table 2]**

| **FACS (%)** | **IFN-gamma (Th1, CD4+)** | **IL-10 (Th2, CD4+)** | **Treg (CD25+/ Foxp3)** | **IFN-gamma - IL-10** | **IFN-gamma - Treg** | **Combined Score** |
|---|---|---|---|---|---|---|
| **SEQ ID NO: 3** | 24.00 | 2.34 | 8.84 | 21.66 | 15.16 | 20.36 |
| **SEQ ID NO: 8** | 16.90 | 5.25 | 8.22 | 11.65 | 8.68 | 11.06 |
| **SEQ ID NO: 10** | 21.00 | 2.03 | 7.66 | 18.97 | 13.34 | 17.84 |
| **SEQ ID NO: 11** | 17.20 | 5.49 | 8.23 | 11.71 | 8.97 | 11.16 |
| **SEQ ID NO: 15** | 17.80 | 4.93 | 7.93 | 12.87 | 9.87 | 12.27 |
| **SEQ ID NO: 17** | 23.20 | 1.93 | 8.17 | 21.27 | 15.03 | 20.02 |
| **SEQ ID NO: 18** | 22.90 | 2.10 | 8.01 | 20.80 | 14.89 | 19.62 |
| **SEQ ID NO: 20** | 16.10 | 5.28 | 8.07 | 10.82 | 8.03 | 10.26 |
| **SEQ ID NO: 22** | 16.90 | 5.15 | 7.65 | 11.75 | 9.25 | 11.25 |
| **SEQ ID NO: 24** | 17.10 | 4.75 | 7.10 | 12.35 | 10.00 | 11.88 |
| **Negative control** | 1.23 | 2.64 | 0.13 | -1.41 | 1.10 | -0.91 |
| **Positive control** | 17.40 | 6.14 | 10.30 | 11.26 | 7.10 | 10.43 |

The results of each FACS analysis above and the scoring results (Combined Score) based on these results could be confirmed through Table 2 above.

As could be confirmed above, the TROP2 immunogenic peptides targeted in the present invention showed excellent effects in that they stimulated IFN-gamma secretion, did not stimulate IL-4 secretion, did not stimulate IL-10 secretion, and did not stimulate regulatory T cells to the maximum.

In other words, the peptides of the present invention exhibited the effect of stimulating Type 1 helper T cells, while not stimulating Type 2 helper T cells and regulatory T cells, and in particular, this effect was most excellent in SEQ ID NOs: 3, 10, 17, and 18.

### Example 3. Confirmation of Type 1 helper T cell-specific immunogenic peptides through ELISPOT analysis

Type 1 helper T cell-specific immunogenic peptides were confirmed through ELISPOT analysis.

### - Experimental method using PBMC

The frequency of interferon-gamma (IFN-γ, 3420-4HPW-10, MabTech, Stockholm, Sweden) and interleukin-10 (IL-10, 3430-4HPW-10, MabTech)-forming T cells was assessed using the ELISpot assay.

Specifically, the pre-coated plates were washed four times with 200 µL/well of PBS. Human PBMCs (PCS-800-011, ATCC, Virginia, USA) 2 x 10⁶/mL were dispensed onto the coated plate, and TROP2 peptides were dispensed at 10 µg/mL each and reacted with the PBMCs. The plate reacted under a total volume of 200 µL/well was incubated at 5% CO₂, 37°C for 70-72 hours. Then, the plate was washed 5 times with 200 µL of PBS, and 200 µL of diluted detection antibody (IFN-γ:7-B6-1-biotin; IL-10:12G8-biotin) was added to the wells of the plate, and then reacted at room temperature for 2 hours. After the reaction, the plate was washed 5 times with 200 µL of PBS, and 1:1000 diluted streptavidin-HRP was added at 100 µL/well, followed by reaction at room temperature for 1 hour. Then, the resulting product was washed 5 times with 200 µL of PBS, 100 µL/well of TMB substrate solution was added and reacted at RT for up to 5-10 minutes, and then the spots were counted using the AID iSpot reader system (AID, Strassberg, Germany).

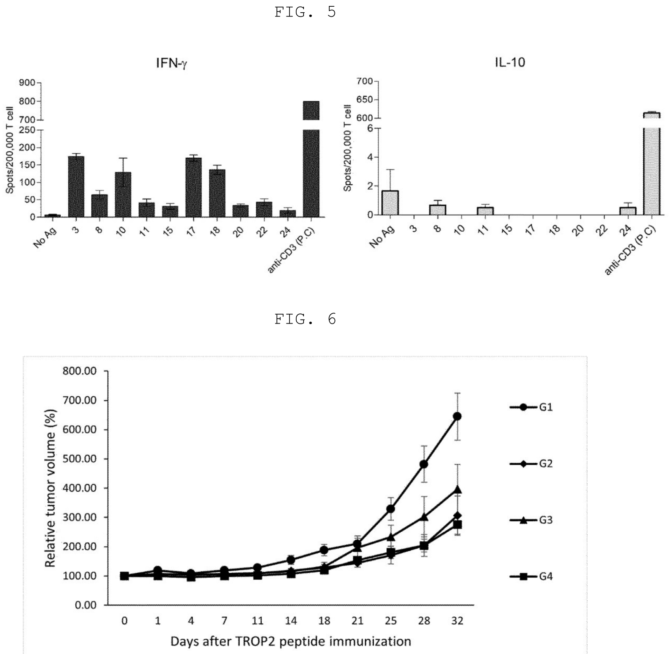

As could be confirmed in FIG. 5, most of the peptides increased the number of cells secreting IFN-γ, but did not increase the number of IL-10 secreting cells, and in particular, the TROP2 immunogenic peptides of SEQ ID NOs: 3, 10, 17, and 19 dramatically increased the secretion of IFN-γ.

### Example 4. Confirmation of antitumor effect of cancer vaccine alone in Xenograft cancer mouse model

Five-week-old athymic Balb/C nude mice weighing 16 to 18 g were purchased and acclimated for at least 3 to 7 days.

The MDA-MB-231 cell line, a human-derived triple-negative breast cancer cell line, was suspended at 5 x 10⁶ cells per individual in 200 µl of a 1:1 mixture of PBS and Matrigel and injected subcutaneously into the axillary region between the right scapular area and the thoracic wall of the mouse.

On Day 16 after MDA-MB-231 cell transplantation, 32 mice (mean tumor size 143.8 ± 2.9 mm³) with tumor sizes reaching approximately 150 mm³ were selected and assigned to each group of 8 mice.

Each group was divided into Group 1 (G1): PBS + CFA/IFA, Group 2 (G2): SEQ ID NO: 3 peptide + CFA/IFA + mGM-CSF, Group 3 (G3): SEQ ID NO: 10 peptide + CFA/IFA + mGM-CSF, and Group 4 (G4): SEQ ID NO: 17 peptide + CFA/IFA+mGM-CSF.

Next, the immunogenic peptide (TROP2 peptide) according to the present invention was administered subcutaneously at a dose of 100 ug per mouse on days 1, 8, 15, and 22 (the 16th day after cell transplantation was designated as Day 0, and the following day was designated as Day 1) for a total of 4 times, and the tumor size for each individual was calculated 11 times (0, 1, 4, 7, 11, 14, 18, 21, 25, 28, and 32) from Day 0 to Day 32. Then, the tumor growth change rate was calculated by comparison with the control group of each Test Group. The analysis results are shown in Table 3 and FIG. 6.

**[Table 3]**

| **Group** | **Treatment** | **TGI rate (%) at Day 32** |
|---|---|---|
| **G1** | PBS+CFA/IFA | - |
| **G2** | Peptide (SEQ ID NO: 3) + CFA/IFA + mGM-CSF | 52.4 |
| **G3** | Peptide (SEQ ID NO: 10) + CFA/IFA + mGM-CSF | 38.5 |
| **G4** | Peptide (SEQ ID NO: 17) + CFA/IFA + mGM-CSF | 57.2 |

As could be confirmed in Table 3 and FIG. 6, the antitumor effects (tumor size and tumor growth inhibition ratio) of the immunogenic peptides alone against the selected TROP2 cancer antigen showed significant antitumor effects for all peptides when compared to the control group (G1).

Further, it was also confirmed through experiments on the xenograft cancer mouse model under the same conditions that when three immunogenic peptides of SEQ ID NOs: 3, 10, and 17 were mixed, a synergistic effect was observed compared to each of the immunogenic peptides.

### Example 5. Confirmation of antitumor effect of cancer vaccine and antibody-drug conjugate (ADC) in Xenograft cancer mouse model

In order to confirm the antitumor effect of the combination therapy of the immunogenic peptide discovered in the present invention and antibody-drug conjugate (ADC), the antitumor effect was analyzed for the following groups using the same experimental method as in Example 4.

Group 1 (G1): PBS+CFA/IFA, Group 2 (G2): SEQ ID NOs: 3, 10, and 17 Peptide Mix + CFA/IFA + mGM-CSF, Group 3 (G3): SEQ ID NOs: 3, 10, 17 Peptide Mix + CFA/IFA + Mgm-CSF + Trodelvy, Group 4 (G4): Trodelvy

The immunogenic peptide (TROP2 peptide) according to the present invention was administered subcutaneously four times in total on the same schedule as Example 4. Trodelvy was administered intravenously twice in total on Days 4 and 11 at a dose of 0.81 mg/kg per mouse.

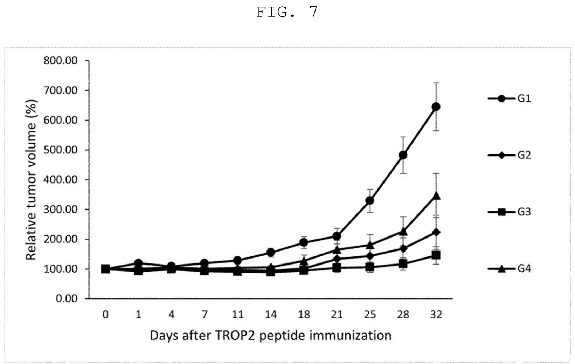

The analysis results are shown in Table 4 and FIG. 7.

**[Table 4]**

| **Group** | **Treatment** | **TGI rate (%) at Day 32** |
|---|---|---|
| **G1** | PBS+CFA/IFA | |
| **G2** | Peptide Mix (SEQ ID NOs: 3, 10, and 17) + CFA/IFA + mGM-CSF | 65.4 |
| **G3** | Peptide Mix (SEQ ID NOs: 3, 10, and 17) + CFA/IFA+mGM-CSF + Trodelvy | 77.4 |
| **G4** | Trodelvy | 46.1 |

As could be confirmed in Table 4 and FIG. 7, not only was the antitumor effect of TROP2 immunogenic peptide mix superior to the relatively low dose of Trodelvy, but also the highest antitumor effect was observed when the TROP2 immunogenic peptide mix and low dose of Trodelvy were combined.

Therefore, the TROP2 immunogenic peptide of the present invention has an antitumor effect as an individual peptide itself, and the combination of two or more of the immunogenic peptides not only exhibits a synergistic effect, but also shows a strong antitumor effect when combined with low dose Trodelvy. In conclusion, the TROP2 immunogenic peptide of the present invention can be considered to not only treat or prevent tumors by itself, but also has the potential to maintain its antitumor effect even when the standard dose of the toxic anticancer agent is reduced when combined with other anticancer agents.

While the foregoing has described in detail certain aspects of the present invention, it will be apparent to one of ordinary skill in the art that these specific descriptions are merely preferred embodiments and are not intended to limit the scope of the invention. Therefore, the substantive scope of the invention will be defined by the appended claims and equivalents thereof.

## Claims

1. An immunogenic peptide, isolated from the TROP2 full-length peptide of SEQ ID NO: 1, having a sequence of 50 or fewer amino acids, and comprising any one amino acid sequence selected from the group consisting of SEQ ID NO: 2 to SEQ ID NO: 27.

2. The immunogenic peptide of claim 1, wherein the immunogenic peptide has a sequence of 15 to 34 consecutive amino acids based on the peptide of SEQ ID NO: 1.

3. The immunogenic peptide of claim 1, wherein the immunogenic peptide has at least 90% homology to any one amino acid sequence selected from the group consisting of SEQ ID NO: 2 to SEQ ID NO: 27.

4. The immunogenic peptide of claim 1, wherein the immunogenic peptide comprises any one sequence selected from the group consisting of SEQ ID NOs: 3, 8, 10, 11, 15, 17, 18, 20, 22, and 24.

5. An immunogenic composition comprising:
at least one peptide according to any one of claims 1 to 4; and
one or more pharmaceutically acceptable carriers.

6. The immunogenic composition of claim 5, further comprising:
a preservative or excipient.

7. The immunogenic composition of claim 5, further comprising:
an adjuvant.

8. A pharmaceutical composition for preventing or treating cancer, comprising:
at least one peptide according to any one of claims 1 to 4.

9. The pharmaceutical composition of claim 8, further comprising:
an additional cancer therapeutic agent.

10. The pharmaceutical composition of claim 9, wherein the additional cancer therapeutic agent is at least one selected from the group consisting of trastuzumab deruxtecan (Enhertu), sacituzumab govitecan (Trodelvy), brentuximab vedotin (Adcetris), trastuzumab emtansine (Kadcyla), polatuzumab vedotin (Polivy), inotuzumab ozogamicin (Besponsa), gemtuzumab ozogamicin (Mylotarg), and moxetumomab pasudotox (Lumoxiti).

11. The pharmaceutical composition of claim 9, wherein the additional cancer therapeutic agent is sacituzumab govitecan (Trodelvy).

12. The pharmaceutical composition of claim 8, further comprising an adjuvant.

13. The pharmaceutical composition of claim 8, wherein the cancer is any one selected from the group consisting of breast cancer, brain cancer, nerve cancer, colorectal cancer, lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, perianal cancer, colon cancer, breast cancer, fallopian tube carcinoma, endometrial carcinoma, cervical cancer, vaginal cancer, vulvar carcinoma, Hodgkin's disease, esophageal cancer, small intestinal cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary CNS lymphoma, spinal cord tumor, brainstem glioma, and pituitary adenoma.

14. An anticancer vaccine composition comprising at least one peptide according to any one of claims 1 to 4.

15. A method for treating or preventing cancer, comprising:
administering to a subject the immunogenic peptide according to any one of claims 1 to 4; the immunogenic composition according to any one of claims 5 and 7; the pharmaceutical composition according to any one of claims 8 to 13; or the anticancer vaccine composition according to claim 14.

16. The method of claim 15, wherein the cancer is any one selected from the group consisting of breast cancer, brain cancer, nerve cancer, colorectal cancer, lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, perianal cancer, colon cancer, breast cancer, fallopian tube carcinoma, endometrial carcinoma, cervical cancer, vaginal cancer, vulvar carcinoma, Hodgkin's disease, esophageal cancer, small intestinal cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary CNS lymphoma, spinal cord tumor, brainstem glioma, and pituitary adenoma.

17. Use in the manufacture of a medicament for treating or preventing cancer, of the immunogenic peptide according to any one of claims 1 to 4; the immunogenic composition according to any one of claims 5 and 7; the pharmaceutical composition according to any one of claims 8 to 13; or the anticancer vaccine composition according to claim 14.

18. The use of claim 17, wherein the cancer is any one selected from the group consisting of breast cancer, brain cancer, nerve cancer, colorectal cancer, lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, perianal cancer, colon cancer, breast cancer, fallopian tube carcinoma, endometrial carcinoma, cervical cancer, vaginal cancer, vulvar carcinoma, Hodgkin's disease, esophageal cancer, small intestinal cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary CNS lymphoma, spinal cord tumor, brainstem glioma, and pituitary adenoma.

19. A pharmaceutical composition for use in treating or preventing cancer, comprising the immunogenic peptide according to any one of claims 1 to 4.

20. The pharmaceutical composition of claim 19, wherein the cancer is any one selected from the group consisting of breast cancer, brain cancer, nerve cancer, colorectal cancer, lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, perianal cancer, colon cancer, breast cancer, fallopian tube carcinoma, endometrial carcinoma, cervical cancer, vaginal cancer, vulvar carcinoma, Hodgkin's disease, esophageal cancer, small intestinal cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary CNS lymphoma, spinal cord tumor, brainstem glioma, and pituitary adenoma.

21. Use in the manufacture of an anticancer vaccine composition, of the immunogenic peptide according to any one of claims 1 to 4; the immunogenic composition according to any one of claims 5 and 7; the pharmaceutical composition according to any one of claims 8 to 13; or the anticancer vaccine composition according to claim 14.

22. An anticancer vaccine composition comprising the immunogenic peptide according to any one of claims 1 to 4 for use in an anticancer vaccine.
